# EUROPÄISCHE PATENTSCHRIFT

(11) **EP 1 322 420 B1**
(45) Veröffentlichungstag und Bekanntmachung des Hinweises auf die Patenterteilung: **09.04.2008**
(21) Anmeldenummer: 01978378.6
(22) Anmeldetag: 20.09.2001
(51) Int. Cl.: B01J 38/52, C07C 209/48

(54) **VERFAHREN ZUR REGENERIERUNG VON KATALYSATOREN**
METHOD FOR REGENERATING CATALYSTS
PROCEDE DE REGENERATION DE CATALYSEURS

(30) Priorität: 25.09.2000 DE 10047703
(43) Veröffentlichungstag der Anmeldung: 02.07.2003
(73) Patentinhaber: BASF SE, 67056 Ludwigshafen (DE)
(72) Erfinder: OHLBACH, Frank, 40219 Düsseldorf (DE); LUYKEN, Hermann, 67069 Ludwigshafen (DE); ANSMANN, Andreas, 69168 Wiesloch (DE); FISCHER, Rolf-Hartmuth, 69121 Heidelberg (DE); MELDER, Johann-Peter, 67459 Böhl-Iggelheim (DE); BASSLER, Peter, 68519 Viernheim (DE)
(86) Internationale Anmeldenummer: PCT/EP2001/010849
(87) Internationale Veröffentlichungsnummer: WO 2002/024334

(56) Entgegenhaltungen:
- EP-A- 0 521 716
- WO-A-99/33561
- US-A- 4 322 315

## Beschreibung

Die vorliegende Erfindung betrifft ein Verfahren zur Regenerierung eines zur Herstellung von NH₂-Gruppen enthaltenden Verbindungen durch Hydrierung von Verbindungen, die mindestens eine ungesättigte Kohlenstoff-Stickstoff-Bindung enthalten, mit Wasserstoff eingesetzten heterogenen Katalysators, dadurch gekennzeichnet, daß man
a) die Zufuhr der zu hydrierenden Verbindung stoppt, und
b) den heterogenen Katalysator mit einer Verbindung der Formel

   R¹ R² N - CO - R³ (I),

   wobei
   - R¹:: Wasserstoff, C₁-C₄-Alkyl,
   - R², R³: unabhängig voneinander jeweils Wasserstoff, C₁-C₄-Alkyl, oder zusammen eine C₃-C₆-Alkylen-Gruppe,
bedeuten,
oder deren Gemische bei einem Druck im Bereich von 0,1 bis 30 MPa und einer Temperatur im Bereich von 100 bis 300°C behandelt mit der Maßgabe, daß die Verbindung der Formel (I) bei der Behandlung flüssig vorliegt.

Verfahren zur Herstellung von NH₂-Gruppen enthaltenden Verbindungen durch Hydrierung von Verbindungen, die mindestens eine ungesättigte Kohlenstoff-Stickstoff-Bindung enthalten, mit Wasserstoff in Gegenwart eines heterogenen Katalysators sind allgemein bekannt, beispielsweise aus Houben-Weyl, Bd. 11/1 (Stickstoff-Verbindungen II, Amine), S. 545-574, 4. Auflage, 1957.

Aus WO 97/37963, WO 97/37964, WO 98/04515 und DE-A-198 09 688 sind Verfahren zur Hydrierung von Adipodinitril zu Hexamethylendiamin oder Mischungen enthaltend 6-Aminocapronitril und Hexamethylendiamin in Gegenwart heterogener Katalysatoren, die auf Eisen, Cobalt oder Nickel oder deren Verbindungen basieren, bekannt.

In solchen Verfahren verschlechtern sich relevante Katalysatoreigenschaften, wie Aktivität oder Selektivität, im Laufe der Betriebszeit.

Um ein Absinken der Aktivität zu kompensieren, kann zwar die Reaktionstemperatur angehoben werden. Nachteilig hierbei ist, daß mit einer Erhöhung der Reaktionstemperatur üblicherweise die Selektivität eines Katalysators fällt und zudem die höhere Temperatur das weitere Absinken der Aktivität beschleunigt.

Es sind Verfahren bekannt, um einen Katalysator, dessen relevante Eigenschaften, wie Aktivität oder Selektivität, sich im Laufe der Betriebszeit verschlechtert haben, zu regenerieren. Unter Regenerierung wird dabei ein Verfahren verstanden, durch das die genannten Katalysatoreigenschaften wieder verbessert werden. So beschreiben WO 97/37963, WO 97/37964 und WO 98/04515 Verfahren zur Regenerierung eines zur Herstellung von NH₂-Gruppen enthaltenden Verbindungen durch Hydrierung von Verbindungen, die mindestens eine ungesättigte Kohlenstoff-Stickstoff-Bindung enthalten, mit Wasserstoff eingesetzten heterogenen Katalysators, dessen Aktivität und Selektivität abgesunken war, durch eine Behandlung mit Wasserstoff unter definierten Verfahrensbedingungen.

Diese Regenerierverfahren führen zwar zu einem guten Erfolg hinsichtlich der Verbesserung der Selektivität und Aktivität. Wünschenswert wäre allerdings eine Absenkung der zur Regenerierung erforderlichen Temperatur und Zeit, um den Katalysator zu schonen.

Der vorliegenden Erfindung lag die Aufgabe zugrunde, ein Verfahren zur Regenerierung eines zur Herstellung von NH₂-Gruppen enthaltenden Verbindungen durch Hydrierung von Verbindungen, die mindestens eine ungesättigte Kohlenstoff-Stickstoff-Bindung enthalten, mit Wasserstoff eingesetzten heterogenen Katalysators zur Verfügung zu stellen, das die Regenerierung auf technisch einfache und wirtschaftliche Weise ermöglicht unter Vermeidung der genannten Nachteile.

Demgemäß wurde das eingangs definierte Verfahren gefunden.

Die zu regenerierenden heterogenen Katalysatoren wurden zuvor zur Herstellung von NH₂-Gruppen enthaltenden Verbindungen durch Hydrierung von Verbindungen, die mindestens eine ungesättigte Kohlenstoff-Stickstoff-Bindung enthalten, mit Wasserstoff eingesetzt.

Als Verbindungen, die mindestens eine ungesättigte Kohlenstoff-Stickstoff-Bindung, wie eine Kohlenstoff-Stickstoff-Doppel- oder Dreifachbindung, setzt man bevorzugt ein C₄-C₈-Alkylnitril oder -dinitril oder Gemische solcher Nitrile oder Dinitrile ein, wie Butannitril, Pentannitril, Hexannitril, Heptannitril, Oktannitril, Butandinitril, Pentandinitril, Hexandinitril, Heptandinitril, Oktandinitril, besonders bevorzugt endständige C₄-C₈-Dinitrile, wie 1,4-Dicyanobutan ("Adipodinitril"), 1,5-Dicyanopentan, 1,6-Dicyanohexan, 1,7-Dicyanoheptan und 1,8-Dicyanooctan, C₅-C₈-Cycloalkylnitrile oder -dinitrile, wie Cyclopentylnitril, Cyclohexylnitril, Cycloheptylnitril, Cyclooctylnitril, Cyclopentyldinitril, Cyclohexyldinitril, Cycloheptyldinitril, Cyclooctyldinitril, oder 4 bis 8 Kohlenstoffatome enthaltende Aminonitrile, bevorzugte alpha, omega-Aminonitrile, wie 5-Aminovaleronitril und 6-Aminocapronitril ein.

Die Nitrile, Dinitrile und Aminonitrile können auch andere funktionelle Gruppen tragen soweit diese die Hydrierung nicht beeinträchtigen, oder gleichzeitige oder teilweise Hydrierung gewünscht ist. Beispielhaft seien C₁-C₄-Alkylgruppen, Arylgruppen, insbesondere Phenyl, C₅-C₈-Cycloalkyl, Aminoalkyl, N-Alkyl-aminoalkyl, N-(Cyanomethyl)aminoalkyl und die Iminogruppe (C=NH, C=NR) genannt, vorzugsweise die Iminogruppe.

Insbesondere kommen Adipodinitril, 6-Aminocapronitril, 3-Cyano-3,5,5-trimethylcyclohexylimin, NC-(CH₂)₂-NH-(CH₂)₂-CN, NC-(CH₂)₂-NH-(CH₂)₂-NH-(CH₂)₂-CN und 1-Cyano-2-aminoethan, insbesondere bevorzugt Adipodinitril in Betracht.

In einer vorteilhaften Ausführungsform kann Adipodinitril zu Hexamethylendiamin hydriert werden.

In einer weiteren vorteilhaften Ausführungsform kann Adipodinitril zu einer Mischung enthaltend Hexamethylendiamin und 6-Aminocapronitril hydriert werden.

Als zu regenerierende heterogene Katalysatoren kommen nach bisherigen Beobachtungen hinsichtlich der katalytisch aktiven Komponente alle für die zur Herstellung von NH₂-Gruppen enthaltenden Verbindungen durch Hydrierung von Verbindungen, die mindestens eine ungesättigte Kohlenstoff-Stickstoff-Bindung enthalten, mit Wasserstoff bekannten heterogenen Katalysatoren in Betracht.

In einer bevorzugten Ausführungsform kann man einen heterogenen Katalysator einsetzen, der als katalytisch aktive Komponente ein, Element ausgewählt aus der Gruppe bestehend aus Eisen, Cobalt und Nickel oder deren Gemische, besonders bevorzugt Eisen oder Cobalt oder deren Gemische, insbesondere Eisen, enthält.

In einer weiteren bevorzugten Ausführungsform kann man einen heterogenen Katalysator einsetzen, der als katalytisch aktive Komponente eine Verbindung eines Elements ausgewählt aus der Gruppe bestehend aus Eisen, Cobalt und Nickel oder deren Gemische, besonders bevorzugt Eisen oder Cobalt oder deren Gemische, insbesondere Eisen enthält.

Solche Katalysatoren kann man ohne Träger, insbesondere für Festbett- oder Suspensionsfahrweise, beispielsweise in Form von Raney-Katalysatoren oder anderen ungeträgerten Formen (sogenannte Vollkontakt-Katalysatoren) einsetzen. Die ungeträgerte Formen können im Vergleich zum hohen Gehalt an aktiver Komponente geringe Beimengungen enthalten. Diese Beimengungen können günstige Auswirkungen auf entweder die katalytische Aktivität und/oder Selektivität, oder auch auf die Eigenschaften wie Härte, Abrieb, chemische oder thermische Beständigkeit des Katalysators haben. Die Gesamtmenge der Beimengungen liegt im allgemeinen im Bereich von 0 bis 20 Gew.-%, bezogen auf die Menge der aktiven Komponente. Als Beimengungen kann man Oxide, Phosphate und Sulfate der Alkalimetall- und Erdalkalimetallverbindungen, thermisch stabile Oxide, wie Siliziumdioxid, Aluminiumoxid, Titandioxid und Zirkondioxid sowie andere Übergangsmetalloxide einsetzen. Ein Einsatz als Trägerkatalysator ist ebenfalls möglich. Als Trägermaterial kommen allgemein keramische Träger, wie Aluminiumoxid, Siliziumdioxid, Titandioxid, Zirkondioxid, oder Siliziumcarbid oder Aktivkohle in Betracht. Bei geträgerten Katalysatoren liegt der Gehalt an aktiver Komponente zu Träger in der Regel im Bereich von 3 bis 95, bevorzugt 30 bis 95 Gew.-%.

Die Katalysatoren kann man auch, wenn gewünscht, mit Metallen der Gruppe VIB (Cr, Mo, W), VIII des Periodensystems der Elemente (Ru, Os, Rh, Ir, Pd, Pt) sowie Kupfer, Mangan und Rhenium modifizieren, wobei der Gehalt an dem nicht-modifizierenden Anteil der aktiven Komponente, bezogen auf die gesamte aktive Komponente, im allgemeinen im Bereich von 50 bis 99,9, bevorzugt von 80 bis 99 Gew.-% beträgt.

Des weiteren kann man die Katalysatoren mit einer Verbindung auf der Basis eines Alkalimetalls oder Erdalkalimetalls wie Lithium, Natrium, Kalium, Rubidium, Cäsium, Magnesium, Calcium, Strontium und Barium, modifizieren. Üblicherweise wählt man ein Gewichtsverhältnis im Bereich von 0 bis 5, bevorzugt 0,1 bis 3 Gew.-% Alkalimetall bzw- Erdalkalimetall, bezogen auf den nicht-modifizierenden Anteil der aktiven Komponente.

Des weiteren kann man die Katalysatoren im Falle von solchen Katalysatoren, deren aktive Komponente auf Eisen oder einer Eisenverbindung basieren, mit einer Verbindung auf der Basis von 1, 2, 3, 4 oder 5 Elementen ausgewählt aus der Gruppe bestehend aus Aluminium, Silizium, Zirkonium, Titan und Vanadium modifizieren. Üblicherweise wählt man ein Gewichtsverhältnis 0 bis 3 Gew.-% Modifizierungsmittel, bezogen auf Eisen.

Des weiteren kann man die Katalysatoren im Falle von solchen Katalysatoren, deren aktive Komponente auf Eisen oder einer Eisenverbindung basieren, mit einer Verbindung auf der Basis Mangan modifizieren. Üblicherweise wählt man eine Mangankonzentration von 0 bis 25, vorzugsweise 0,01 bis 5 Gew.-%, bezogen auf Eisen.

Solche Katalysatoren und deren Herstellung sind an sich bekannt, beispielsweise aus WO 97/37963, WO 97/37964, WO 98/04515 und DE-A-198 09 688.

Vor dem Einsatz können die Katalysatoren aktiviert werden, beispielsweise durch eine Behandlung mit Wasserstoff, wobei man die Aktivierung drucklos oder bei erhöhtem Druck bei Temperaturen ab 200°C in an sich bekannter Weise durchführen kann.

Die Hydrierung kann man in Sumpf- Riesel oder Suspensionsfahrweise durchführen.

Führt man die Hydrierung in Suspension durch, wählt man üblicherweise Temperaturen im Bereich von 40 bis 150°C, vorzugsweise von 50 bis 100, besonders bevorzugt von 60 bis 90°C; den Druck wählt man im allgemeinen im Bereich von 2 bis 20, vorzugsweise von 3 bis 10, besonders bevorzugt von 4 bis 9 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise im Bereich von 50 bis 275, vorzugsweise von 70 bis 200 min.

Bei der Suspensionsfahrweise kann man zusätzlich ein flüssiges Verdünnungsmittel zusetzen. Als flüssiges Verdünnungsmittel kommen bevorzugt Ammoniak, Amine, Diamine und Triamine mit 1 bis 12 Kohlenstoffatomen wie Trimethylamin, Triethylamin, Tripropylamin, Tributylamin, oder Alkohole, insbesondere Methanol oder Ethanol, besonders bevorzugt Ammoniak in Betracht. Zweckmäßig wählt man die Konzentration an zu hydrierender Verbindung im Bereich von 10 bis 90, vorzugsweise von 30 bis 80, besonders bevorzugt von 40 bis 70 Gew.-%, bezogen auf die Summe von zu hydrierender Verbindung und flüssigem Verdünnungsmittel.

Die Menge an Katalysator wählt man im allgemeinen so, daß die Katalysator-Menge im Bereich von 1 bis 50, bevorzugt von 5 bis 20 Gew.-%, bezogen auf die Menge an zu hydrierender Verbindung, beträgt.

Die Suspensionshydrierung kann man diskontinuierlich oder kontinuierlich, bevorzugt kontinuierlich, in der Regel in der Flüssigphase durchführen.

Man kann die Hydrierung auch diskontinuierlich oder kontinuierlich, vorzugsweise kontinuierlich in eine Festbettreaktor in Riesel- oder Sumpffahrweise durchführen, wobei man üblicherweise eine Temperatur im Bereich von 30 bis 200, vorzugsweise von 50 bis 150°C, und einen Druck in der Regel im Bereich von 2 bis 30, vorzugsweise 3 bis 20 MPa wählt. Bevorzugt führt man die Hydrierung in Gegenwart eines flüssigen Verdünnungsmittels, bevorzugt Ammoniak, Amine, Diamine und Triamine mit 1 bis 12 Kohlenstoffatomen wie Trimethylamin, Triethylamin, Tripropylamin, Tributylamin, oder Alkohole, insbesondere Methanol oder Ethanol, besonders bevorzugt Ammoniak, durch.

In einer bevorzugten Ausführungsform wählt man einen Gehalt an Ammoniak im Bereich von 0,5 bis 10, bevorzugt von 1 bis 6 g pro Gramm zu hydrierender Verbindung, insbesondere Adipodinitril.

Bevorzugt wählt man dabei eine Katalysatorbelastung im Bereich von 0,1 bis 2,0, vorzugsweise von 0,3 bis 1,0 kg der zu hydrierenden Verbindung / l*h, insbesondere Adipodinitril / l*h. Auch hier kann man durch Veränderung der Verweilzeit den Umsatz und damit die Selektivität gezielt einstellen.

Die Hydrierung kann man in einem üblichen hierfür geeigneten Reaktor durchführen.

Führt man die Umsetzung in der Gasphase durch, wählt man üblicherweise Temperaturen im Bereich von 100 bis 250°C, vorzugsweise von 160 bis 200°C; den Druck wählt man im allgemeinen im Bereich von 0,01 bis 3, vorzugsweise 0,09 bis 0,5 MPa. Des weiteren setzt man in der Regel von 2 bis 300, bevorzugt von 10 bis 200 Mol Wasserstoff pro Mol Verbindung, die mindestens eine ungesättigte Kohlenstoff-Stickstoff-Bindung enthält, ein.

In einer bevorzugten Ausführungsform führt man die Hydrierung der zu hydrierenden Verbindung, insbesondere Adipodinitril, in Gegenwart von Ammoniak als flüssigem Verdünnungsmittel mit Festbettkatalysatoren durch.

Zur Regenerierung des Katalysators stoppt man zunächst den Zulauf an zu hydrierender Verbindung, insbesondere Adipodinitril, und, soweit eingesetzt, flüssigem Verdünnungsmittel. Vorteilhaft kann man den Katalysator und das Reaktionsgemisch trennen.

Die Zufuhr an Wasserstoff kann ebenfalls gestoppt werden, vorteilhaft kommt eine Regenerierung in Gegenwart von Wasserstoff in Betracht.

Erfindungsgemäß behandelt man den zu regenierenden Katalysator mit einer Verbindung der Formel (I)

R¹ R² N - CO - R³ (I),

oder deren Gemische.

In dieser Formel steht R¹ für Wasserstoff oder eine C₁-C₄-Alkyl, wie Methyl, Ethyl, n-Propyl, i-Propyl, n-Butyl, i-Butyl, s-Butyl, t-Butyl, vorzugsweise Wasserstoff oder Methyl.

R², R³ stehen unabhängig voneinander jeweils für Wasserstoff, C₁-C₄-Alkyl, wie Methyl, Ethyl, n-Propyl, i-Propyl, n-Butyl, i-Butyl, s-Butyl, t-Butyl, vorzugsweise Wasserstoff oder Methyl, oder zusammen für eine C₃-C₆-Alkylen-Gruppe der Art -(CH₂)ₙ-, in der n die Werte 3, 4, 5 oder 6, vorzugsweise 3, annehmen kann.

Die Gruppen R¹, R² und R³ können für den Fall, daß sie nicht Wasserstoff darstellen, Substituenten, wie Arylgruppen, insbesondere Phenyl, C₅-C₈-Cycloalkyl, Aminoalkyl und, falls die betreffende Gruppe R¹, R² oder R³ nicht Methyl ist, C₁-C₄-Alkylgruppen sein.

Die Gruppen R¹, R² und R³ können für den Fall, daß sie nicht Wasserstoff darstellen, funktionelle Gruppen, wie Halogene, beispielsweise Fluor, Chlor, Brom, tragen.

Vorzugsweise tragen die Gruppen R¹, R² und R³ keine funktionellen Gruppen.

In einer bevorzugten Ausführungsform stehen R² und R³ zusammen für eine C₃-Alkylen-Gruppe der Art -(CH₂)₃₋.

In einer weiteren bevorzugten Ausführungsform stehen R² und R³ zusammen für eine C₃-Alkylen-Gruppe der Art -(CH₂)₃- und R¹ für eine Methylgruppe.

In einer weiteren bevorzugten Ausführungsform stehen R¹ und R² für jeweils eine Methylgruppe und R³ für Wasserstoff.

Erfindungsgemäß wird der heterogene Katalysator mit einer Verbindung der Formel (I) behandelt mit der Maßgabe, daß die Verbindung der Formel (I) bei der Behandlung flüssig vorliegt.

Unter der Behandlung wird ein diskontinuierliches oder kontinuierliches Inkontaktbringen des Katalysators mit einer Verbindung der Formel (I) verstanden.

Im Falle eines Suspensionskatalysators kann der Katalysator vorteilhaft in einer Verbindung der Formel (I) suspendiert werden. Die Verbindung der Formel (I) kann dem Katalysator kontinuierlich oder diskontinuierlich zugeführt werden. Die Verbindung der Formel (I) kann von dem Katalysator kontinuierlich oder diskontinuierlich abgetrennt werden.

Im Falle eines Festbettkatalysators kann der Katalysator vor der Regenerierung aus dem Reaktor ausgebaut werden. In einer vorteilhaften Ausführungsform kann man die Regenerierung in dem Hydrierreaktor ohne Ausbau des Katalysators vornehmen.

Im Falle eines Festbettkatalysators kann der Katalysator vorteilhaft von einer Verbindung der Formel (I) kontinuierlich oder diskontinuierlich durchströmt werden. Die Verbindung der Formel (I) kann dem Katalysator kontinuierlich oder diskontinuierlich zugeführt werden. Die Verbindung der Formel (I) kann von dem Katalysator kontinuierlich oder diskontinuierlich abgetrennt werden.

Die vom Katalysator abgetrennte Verbindung der Formel (I) kann verworfen oder vorteilhaft, gegebenenfalls nach einer Reinigung, erneut dem Katalysator zugeführt werden.

Erfindungsgemäß führt man die Regenerierung bei einem Druck im Bereich von 0,1 bis 30 MPa durch. Führt man die Regenerierung in Gegenwart von Wasserstoff durch, so kann vorteilhaft die Regenerierung bei dem bei der Hydrierung zuvor angewandte Druck durchgeführt werden. Bei kontinuierlicher Regenerierung kommt vorteilhaft eine Wasserstoffmenge im Bereich von 1 bis 100, vorzugsweise 2 bis 50 g Wasserstoff / 1 Reaktorvolumen * Stunde in Betracht.

Die Regenerierung kann man bei Temperaturen von mindestens 100, vorzugsweise mindestens 120, insbesondere mindestens 140, besonders bevorzugt mindestens 160°C durchführen.

Als obere Temperaturgrenze zur Regenerierung kommt die Zersetzungstemperatur der Verbindung der Formel (I), die im allgemeinen bei 300°C liegt, in Betracht, vorzugsweise höchstens 200°C, insbesondere höchstens 180°C.

Die Dauer der Behandlung des zu regenerierenden Katalysators beträgt im allgemeinen 1 bis 12, vorzugsweise 2 bis 4 Stunden.

Nach der Behandlung trennt man den regenerierten Katalysator und die Verbindung der Formel (I) voneinander. Der regenerierte Katalysator kann dann wie ein frisch hergestellter Katalysator erneut in einem Verfahren zur Herstellung von NH₂-Gruppen enthaltenden Verbindungen durch Hydrierung von Verbindungen, die mindestens eine ungesättigte Kohlenstoff-Stickstoff-Bindung enthalten, mit Wasserstoff eingesetzt werden.

### Beispiele

### Beispiel 1

Drei in Reihe geschaltete Rohrreaktoren (Gesamtlänge 4,5 m, d = 6 mm) wurden mit 142 ml (240 g) eines gemäß Beispiel 1a von DE-A-198 09 688 hergestellten auf Eisen basierenden Katalysators (1,5 bis 3 mm Splitt) befüllt und anschließend drucklos im Wasserstoffstrom (200 1/h) reduziert. Dazu wurde die Temperatur innerhalb von 24Stunden von 70°C auf 340°C angehoben und anschließend 72 Stunden bei 340°C gehalten.

Nach Absenken der Temperatur wurde dem Reaktor ein Gemisch aus 0,66 mol/h Adipodinitril, 365 ml/h Ammoniak und 1,05 mol/h Wasserstoff zugeführt. Die Reaktortemperatur wurde jeweils so eingestellt, daß die 6-Aminocapronitril- und Hexamethylendiamin-Gesamtselektivität bei einem Druck von 25 MPa konstant war. Hierzu wurde die Temperatur zu Beginn auf 94°C eingestellt und im Laufe von 9000 Stunden auf 107°C angehoben.

Anschließend wurden die Zuläufe abgestellt und der Katalysator bei 160°C vier Stunden mit 100 ml/h N-Methyl-alpha-pyrrolidon gespült.

Dann wurde zunächst vier Stunden bei 107°C mit 365 ml/h Ammoniak gespült und anschließend die ursprünglichen Zuläufe wieder eingestellt. Die Reaktortemperatur konnte zur Erzielung der vor der Regenerierung erhaltenen Gesamtselektivität auf 94°C abgesenkt werden.

### Beispiel 2

In einem kontinuierlich betriebenen Rohrreaktor wurde die Hydrierung von Adipodinitril zu einem Gemisch aus 6-Aminocapronitril und Hexamethylendiamin bei 70 bar und einer Belastung von 0,22 kg/1_{*}h an Cr-dotiertem Raney-Co-Splitt der Splittgröße 1-3 mm in Gegenwart von Ammoniak als Lösungsmittel durchgeführt.

Nach 80 h Betriebszeit betrug der Adipodinitril-Umsatz 92,8% bei einer mittleren Temperatur über die Reaktorlänge von 37,0°C.

Nach 2004 h Betriebszeit hatte der Katalysator kontinuierlich an Aktivität verloren, so daß der Adipodinitril-Umsatz nur noch 59,9% betrug bei einer mittleren Temperatur über die Reaktorlänge von 4 8 , 5°C .

Zur Regenerierung des Katalysators wurden die Zuläufe an Adipodinitril und Ammoniak gestoppt und für 4 Stunden der Katalysator mit N-Methyl-alpha-pyrrolidon bei einer Belastung von 2 kg/l*h und 150°C gespült. Die Wasserstoffzufuhr von 500 Nl/l*h blieb dabei ebenso wie der Druck von 70 bar erhalten. Nach der Regenerierung wurde abgekühlt und der Reaktor wieder wie im Falle eines frischen Katalysators angefahren.

Nach insgesamt 2112 h Betriebszeit wurde ein Adipodinitril-Umsatz von 91,4% und die anfangs erreichte 6-Aminocapronitril- und Hexametyhlendiamin-Gesamtselektivität bei einer mittleren Temperatur über die Reaktorlänge von 37,3°C erzielt.

## Patentansprüche

1. Verfahren zur Regenerierung eines zur Herstellung von NH₂-Gruppen enthaltenden Verbindungen durch Hydrierung von Verbindungen, die mindestens eine ungesättigte Kohlenstoff-Stickstoff-Bindung enthalten, mit Wasserstoff eingesetzten heterogenen Katalysators, **dadurch gekennzeichnet, daß** man
a) die Zufuhr der zu hydrierenden Verbindung stoppt, und
b) den heterogenen Katalysator mit einer Verbindung der Formel (I)
R¹ R² N - CO - R³ (I),
wobei
R¹: Wasserstoff, C₁-C₄-Alkyl,
R², R³ unabhängig voneinander jeweils Wasserstoff, C₁-C₄-Alkyl, oder zusammen eine C₃-C₆-Alkylen-Gruppe,
bedeuten,
oder deren Gemische bei einem Druck im Bereich von 0,1 bis 30 MPa und einer Temperatur im Bereich von 100 bis 300°C behandelt mit der Maßgabe, daß die Verbindung der Formel (I) bei der Behandlung flüssig vorliegt.

2. Verfahren nach Anspruch 1, wobei man einen heterogenen Katalysator regeneriert, der zur Hydrierung von Adipodinitril eingesetzt wurde.

3. Verfahren nach Anspruch 1, wobei man einen heterogenen Katalysator regeneriert, der zur Hydrierung von Adipodinitril zu Hexamethylendiamin eingesetzt wurde.

4. Verfahren nach Anspruch 1, wobei man einen heterogenen Katalysator regeneriert, der zur Hydrierung von Adipodinitril zu einer Mischung enthaltend Hexamethylendiamin und 6-Aminocapronitril eingesetzt wurde.

5. Verfahren nach den Ansprüchen 1 bis 4, wobei R² und R³ in Formel (I) zusammen eine C₃-Alkylen-Gruppe bilden.

6. Verfahren nach den Ansprüchen 1 bis 4, wobei in Formel (I) R² und R³ zusammen für eine C₃-Alkylen-Gruppe und R¹ für eine Methylgruppe stehen.

7. Verfahren nach den Ansprüchen 1 bis 6, wobei in Formel (I) R¹ und R² für jeweils eine Methylgruppe und R³ für Wasserstoff stehen.

8. Verfahren nach den Ansprüchen 1 bis 7, wobei der heterogene Katalysator als katalytisch aktive Komponente ein Element ausgewählt aus der Gruppe bestehend aus Eisen, Cobalt und Nickel oder deren Gemische enthält.

9. Verfahren nach den Ansprüchen 1 bis 7, wobei der heterogene Katalysator als katalytisch aktive Komponente eine Verbindung eines Elements ausgewählt aus der Gruppe bestehend aus Eisen, Cobalt und Nickel oder deren Gemische enthält.

10. Verfahren nach den Ansprüchen 1 bis 9, wobei man die Regenerierung in Gegenwart von Wasserstoff durchführt.

11. Verfahren nach den Ansprüchen 1 bis 9, wobei man in Schritt a) die Zufuhr des Wasserstoffs stoppt.

## Claims

1. A process for the regeneration of a heterogeneous catalyst used for the preparation of compounds comprising NH₂ groups by the hydrogenation, with hydrogen, of compounds comprising at least one unsaturated carbon-nitrogen bond, wherein
a) the feed of the compound to be hydrogenated is stopped and
b) the heterogeneous catalyst is treated with a compound of formula (I):
R¹ R² N - CO - R³ (I),
in which
R¹ is hydrogen or C₁-C₄ alkyl and
R², R³ independently of one another are each hydrogen or C₁-C₄ alkyl or together are a C₃-C₆ alkylene group,
or mixtures of such compounds, at a pressure ranging from 0.1 to 30 MPa and a temperature ranging from 100 to 300°C, with the proviso that the compound of formula (I) is in liquid form during the treatment.

2. The process according to claim 1 wherein the heterogeneous catalyst regenerated has been used for the hydrogenation of adipodinitrile.

3. The process according to claim 1 wherein the heterogeneous catalyst regenerated has been used for the hydrogenation of adipodinitrile to hexamethylenediamine.

4. The process according to claim 1 wherein the heterogeneous catalyst regenerated has been used for the hydrogenation of adipodinitrile to a mixture comprising hexamethylenediamine and 6-aminocapronitrile.

5. The process according to any of claims 1 to 4 wherein, in formula (I), R² and R³ together form a C₃ alkylene group.

6. The process according to any of claims 1 to 4 wherein, in formula (I), R² and R³ together are a C₃ alkylene group and R¹ is a methyl group.

7. The process according to any of claims 1 to 6 wherein, in formula (I), R¹ and R² are each a methyl group and R³ is hydrogen.

8. The process according to any of claims 1 to 7 wherein the heterogeneous catalyst comprises, as the catalytically active component, an element selected from the group consisting of iron, cobalt and nickel or mixtures thereof.

9. The process according to any of claims 1 to 7 wherein the heterogeneous catalyst comprises, as the catalytically active component, a compound of an element selected from the group consisting of iron, cobalt and nickel or mixtures thereof.

10. The process according to any of claims 1 to 9 wherein the regeneration is effected in the presence of hydrogen.

11. The process according to any of claims 1 to 9 wherein the hydrogen feed is stopped in step a).

## Revendications

1. Procédé de régénération d'un catalyseur utilisé pour la synthèse de composés contenant des groupes NH₂ par hydrogénation de composés qui contiennent au moins une liaison carbone-azote insaturée avec de l'hydrogène, **caractérisé en ce que** :
a) l'on interrompt l'apport du composé à hydrogéner; et
b) l'on traite le catalyseur hétérogène avec un composé de formule (I) .
R¹R²N - CO - R³ (I),
dans laquelle
R¹ désigne l'hydrogène, un radical C₁-C₄-alkyle;
R², R³ désignent respectivement, indépendamment l'un de l'autre, l'hydrogène, un radical C₁-C₄-alkyle
ou, ensemble, un radical C₃-C₆-alkylène,
ou leurs mélanges à une pression de l'ordre de 0,1 à 30 MPa et à une température de l'ordre de 100 à 300°C, étant entendu que le composé de formule (I) est présent sous forme liquide lors du traitement.

2. Procédé selon la revendication 1, dans lequel on régénère un catalyseur hétérogène qui a été utilisé pour l'hydrogénation d'adipodinitrile.

3. Procédé selon la revendication 1, dans lequel on régénère un catalyseur hétérogène qui a été utilisé pour l'hydrogénation d'adipodinitrile en hexaméthylènediamine.

4. Procédé selon la revendication 1, dans lequel on régénère un catalyseur hétérogène qui a été utilisé pour l'hydrogénation d'adipodinitrile en un mélange contenant de l'hexaméthylènediamine et du 6-aminocapronitrile.

5. Procédé selon l'une des revendications 1 à 4, dans lequel R² et R³ dans la formule (I) forment ensemble un groupe C₃-alkylène.

6. Procédé selon l'une des revendications 1 à 4, dans lequel, dans la formule (I), R² et R³ forment ensemble un groupe C₃-alkylène et R¹ désigne un groupe méthyle.

7. Procédé selon l'une des revendications 1 à 6, dans lequel, dans la formule (I), R¹ et R² forment ensemble un groupe méthyle et R³ désigne l'hydrogène.

8. Procédé selon l'une des revendications 1 à 7, dans lequel le catalyseur hétérogène contient, comme composant actif catalytiquement, un élément sélectionné dans le groupe composé du fer, du cobalt et du nickel ou de leurs mélanges.

9. Procédé selon l'une des revendications 1 à 7, dans lequel le catalyseur hétérogène contient comme composant actif catalytiquement, un composé d'un élément sélectionné dans le groupe composé du fer, du cobalt, du nickel ou de leurs mélanges.

10. Procédé selon l'une des revendications 1 à 9, dans lequel on effectue la régénération en présence d'hydrogène.

11. Procédé selon l'une des revendications 1 à 9, dans lequel l'apport d'hydrogène est interrompu dans l'étape a).
